(19) Europäisches Patentamt
European Patent Office
Office européen des brevets

(11) **EP 2 055 329 B1**

(12) **EUROPEAN PATENT SPECIFICATION**

(45) Date of publication and mention
of the grant of the patent:
**09.03.2016 Bulletin 2016/10**

(21) Application number: **07745402.3**

(22) Date of filing: **15.06.2007**

(51) Int Cl.:
**A61M 1/02** *(2006.01)*

(86) International application number:
**PCT/JP2007/062148**

(87) International publication number:
**WO 2007/145328 (21.12.2007 Gazette 2007/51)**

(54) **BLOOD TREATMENT FILTER AND BLOOD TREATMENT CIRCUIT**

BLUTBEHANDLUNGSFILTER UND BLUTBEHANDLUNGSKREISLAUF

FILTRE DE TRAITEMENT DU SANG ET CIRCUIT DE TRAITEMENT DU SANG

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **16.06.2006 JP 2006168134**

(43) Date of publication of application:
**06.05.2009 Bulletin 2009/19**

(73) Proprietor: **Terumo Kabushiki Kaisha
Tokyo 151-0072 (JP)**

(72) Inventors:
• **IMAI, Tadashi
Ashigarakami-gun, Kanagawa 259-0151 (JP)**
• **OHKAWARA, Junichi
Ashigarakami-gun, Kanagawa 259-0151 (JP)**
• **MURAKOSHI, Masahide
Ashigarakami-gun, Kanagawa 259-0151 (JP)**

(74) Representative: **TBK
Bavariaring 4-6
80336 München (DE)**

(56) References cited:
**EP-A1- 0 630 675       JP-A- 03 027 317
JP-A- 04 272 768       JP-A- 06 007 429
JP-A- 2003 052 814     JP-A- 2003 190 276
JP-A- 2006 026 327     US-A- 4 701 267
US-A1- 2001 037 078**

**EP 2 055 329 B1**

**Description**

BACKGROUND OF THE INVENTIION

FIELD OF THE INVENTION

The present invention relates to a blood treatment filter and a blood treatment circuit for preparing a blood component product for blood transfusion.

DESCRIPTION OF RELATED ART

Recently, blood transfusion of blood component products free of leukocytes has been widely used to prevent adverse effects which are caused by leukocytes. Here, removal methods for leukocytes are classified into two groups. One method is to remove leukocytes by filtering respective blood components prepared via centrifugal separation of blood (whole blood product) collected from a donor, through an appropriate filter for the respective blood component. The other method is to remove leukocytes by which a blood component product is prepared by removing leukocytes by filtering a whole blood product and separating the resulting blood product centrifugally.

It has been substantially impossible to prepare a platelet product by using a commercially available filter for a whole blood product because not only leukocytes but also platelets having high adhesion property are simultaneously removed from the whole blood product.

With respect to a method to solve the problem, Japanese Laid-Open Patent Publication No. 2001-129078 (claim 1) discloses a method for removing leukocytes. The method uses a leukocyte removing filter whose rate of an effective filtering area to a thickness of a filtering material is defined. Together, a filtering amount per surface area unit of the filtering material filled in the filter is defined. By using this filter, a whole blood product is filtered to prepare a platelet product.

Further, Japanese Patent Laid-Open Publication No. 2001-198213 (claim 12) discloses a filter for removing leukocytes from a blood component product such as concentrated red blood cell product prepared by a .centrifugal separation. The document describes a filter device which includes: a microaggregate removing filter which is made of a fabric porous medium with a predetermined average fiber diameter and includes hydrophilic basic groups containing at least non-ionic hydrophilic groups and basic groups on the surface of the filter; and a leukocyte removing filter arranged at the down-stream side of the microaggregate removing filter. Herein, the concentrated red blood cell product is used for more than 24 hours after blood collecting. Therefore, aggregates are formed by aggregation of proteins and blood cell components in the product. Since the aggregates are removed by the microaggregate removing filter arranged at the up-stream side, clogging of the leukocyte removing filter arranged at the down-stream side is prevented, in the filter device described in Japanese Patent Laid-Open Publication No. 2001-198213.

However, with respect to the leukocyte removal method disclosed in Japanese Patent Laid-Open Publication No. 2001-198213, there is a problem that it is difficult to keep sufficiently high levels of both leukocyte removal rate and platelet recovery rate for the obtained blood component product (final blood product) in an actual amount of treated blood assumed (max. 620 mL) because a treated blood amount is too small (6 to 14 mL).

Further, by using the filter device disclosed in Japanese Patent Laid-Open Publication No. 2001-198213, the platelet component in the blood product is also removed by the microaggregate removing filter arranged at the up-stream side due to the high adhesiveness of platelets. As a result, there is a problem that the platelet recovery rate in the obtained blood component product (final blood product) becomes to decrease.

US 2001/037078 A1 discloses systems and methods for collecting leukocyte-reduced blood components, including plasma that is free or virtually free of cellular blood species.

US 4 701 267 A discloses a method for removing leukocytes from a leukocyte-containing suspension.

EP 0 630 675 A1 teaches a leukocyte or leukocyte/platelet remover and a filter therefore.

SUMMARY OF THE INVENTION

The present invention has been developed in view of the above-mentioned problem. Herein, it is an object of the present invention to provide a blood treatment filter and a blood treatment circuit in which a blood cell removal rate for the predetermined blood cells such as leukocytes (white blood cells) in a final blood product is in a sufficient level, and in which a recovery rate of the other blood components such as platelets is in an excellent level, even in a treated blood amount assumed for an actual blood component product (max. about 620 mL).

In order to solve the above-mentioned problem, a blood treatment filter of the present invention is defined according to claim 1.

According to the above-mentioned construction, movement of blood cells flowing into the main filter unit is adjusted and stagnation of the influx blood cells is prevented, by providing the pre-filter unit at the up-stream side of the main filter unit capturing leukocytes; the pre-filter having a predetermined permeability rate of micro particles within a predetermined range of a particle size (6 $\mu$m) and not removing the blood components. Hereby, removing of the predetermined blood cell components and passage (recovery) of the remaining blood cell components besides the predetermined blood cell components are going smoothly in the main filter unit.

Further, according to the blood treatment filter of the present invention, when a suspension containing first micro particles with an average particle size of 3 $\mu$m is passed through the main filter unit by gravity, a removal rate of the first micro particles is 85 to 99%.

According to the construction, by determining a removal rate of the first particles (average particle size 3 $\mu$m) for the main filter unit, the removal rate of the predetermined blood cell components is more improved and a recovery rate of the other blood cell components further increases.

Furthermore, according to the blood treatment filter of the present invention, the suspension containing the first particles is passed through the main filter unit by gravity so that an average flow speed is 50 mL/min.

According to the construction, the removal rate of the leukocytes is more improved and the recovery rate of the other blood cell components is further increased because the main filter unit is defined by the removal rate of the first micro particles measured under a specified condition (average flow speed 50 mL/min).

Furthermore, according to the blood treatment filter of the present invention, the suspension containing the second particles having the average particle size of 6 $\mu$m is passed through the pre-filter unit by gravity so that an average flow speed is 50 mL/min.

According to the construction, the flow of the blood cells into the main filter unit is more improved because the permeability rate of the second micro particles for the pre-filter unit measured under a specified condition (average flow speed 50 mL/min) is defined.

Furthermore, according to the blood treatment filter of the present invention, an absolute surface zeta potential value of the pre-filter unit is -2.0 to +2.0 mV.

According to the construction, the flow of blood cells into the main filter unit is more improved, by defining the absolute surface zeta potential value of the pre-filter unit to be within the predetermined range.

Furthermore, according to the blood treatment filter of the present invention, a treated blood amount per unit volume of the filter unit is 10 to 20 mL/cm$^3$, when the blood is passed through the blood treatment filter by gravity.

According to the construction, the removal rate of the predetermined blood cell components is more improved and the recovery rate of the other blood cell components is further increased, by defining the treated blood amount per unit volume of the filter unit.

Furthermore, according to the blood treatment filter of the present invention, a volume ratio between the pre-filter unit and the main filter unit is 1:5 to 1:20.

According to the construction, the removal rate of the predetermined blood cell components is more improved and the recovery rate of the other blood cell components is further increased, by defining the volume ratio between the pre-filter unit and the main filter unit.

Furthermore, according to the blood treatment filter of the present invention, the absolute surface zeta potential value of the main filter unit is -2.0 to +2.0 mV.

According to the construction, the removal rate of the predetermined blood cell components is more improved and the recovery rate of the other blood cell components is further increased, by defining the absolute surface zeta potential value of the main filter unit to be within the predetermined range.

Further, according to the blood treatment filter of the present invention, the main filter unit and the pre-filter unit have a sponge-like porous membrane having a number of micro communication holes communicating one side of the membrane to the other side and having a liquid permeable structure.

According to the construction, the flow of the blood cells into the main filter unit is more improved by making the pre-filter unit of the sponge-like porous membrane. Furthermore, by making the main filter unit of the sponge-like porous membrane, the removal rate of the predetermined blood cell components is more improved and the recovery rate of the other blood cell components is further increased.

Further, according to the blood treatment filter of the present invention, when blood is treated within 24 hours after blood collection from a donor, or when the main filter virtually removes only leukocytes from blood and passes erythrocytes and platelets, the removal rate of predetermined blood cells (leukocytes) is more improved and the recovery rate of the other blood cell components (erythrocytes and platelets) is mostly increased.

The blood treatment circuit of the present invention comprises: the above-mentioned blood treatment filter; a blood collecting apparatus including a primary bag for storing the collected blood, which is connected with the inlet of the blood treatment filter; and a blood treatment apparatus including a secondary bag for collecting the remaining blood components which flow from the outlet and from which the predetermined blood cell components are removed, which is connected with the outlet of the blood treatment filter.

According to the construction, by the blood treatment filter, the predetermined blood cell components are efficiently removed from blood (whole blood product) which is collected by a blood collecting apparatus, and the other blood cell components efficiently flow into a blood treatment apparatus.

Further, according to the blood treatment circuit of the present invention, the blood collecting apparatus comprises: the primary bag; a blood collecting needle; a first tube of which end is connected with the primary bag and the other end is connected with the blood collecting needle, respectively; and a second tube of which end is connected with the primary bag and the other end is connected with the inlet of the blood treatment filter, respectively.

According to the construction, the whole blood product is efficiently collected because the blood collecting apparatus includes the primary bag, the blood collecting needle, the first tube and the second tube.

Further, according to the blood treatment circuit of the present invention, the blood treatment apparatus comprises: the secondary bag, a third tube of which end is connected with the outlet of the blood treatment filter and the other end is connected with the secondary bag, respectively; a third bag for storing blood cell components separated from blood cell components stored in the secondary bag; a forth bag filled with medical liquid beforehand; a forth tube of which end is connected with the secondary bag; a fifth tube of which end is connected with the forth tube and the other end is connected with the third bag; a sixth tube of which end part is connected with the forth tube and the other end is connected with the forth bag, respectively.

According to the construction, the remaining blood cell components which flow out through the blood treatment filter are efficiently collected (preserved), because the blood treatment apparatus includes the secondary bag, the third bag, the forth bag, the third tube, the forth tube, the fifth tube, and the sixth tube.

According to the blood treatment filter and the blood treatment circuit of the present invention, by defining the permeability rate of micro particles for the filter unit, even if a treated blood amount is an amount (max. ca. 620 mL) which is assumed in the actual blood product, it is possible that removal rate of the predetermined blood cell components such as leukocytes, is in the obtained blood cell component product (final blood product) is in a sufficient level. Further, it is possible that the recovery rate of the remaining blood cell components such as platelets is in a sufficient level, even if any collecting loss is occurred in the blood treatment filter or the blood treatment circuit.

Further, according to the blood treatment filter and the blood treatment circuit, by defining the absolute surface zeta potential value, the removal rate of micro particles, the treated blood amount, or the volume ratio of the filter unit to be within

a predetermined range, it is possible to sufficiently satisfy the standard of the final blood product. For example, it is possible to satisfy that a leukocyte removal rate is more than 3. (-Log), a platelet recovery rate is 75% or more, and an erythrocyte recovery rate is 90% or more. Moreover, since it is possible to decrease time for filtering blood (filtration duration time) to be under 45 min, excellent operational efficiency for producing a blood product is achieved.

BRIEF DESCRIPTION OF THE DRAWINGS

FIG. 1 is a plan view of the blood treatment filter in the present invention.
FIG. 2 is an X-X cross sectional view of FIG. 1.
FIG. 3 is a plan view of the blood treatment circuit in the present invention.

DETAILED DESCRIPTION OF THE PREFERRED EMBODIMENTS

The blood treatment filter of the present invention will be described in detail by referring to drawings.

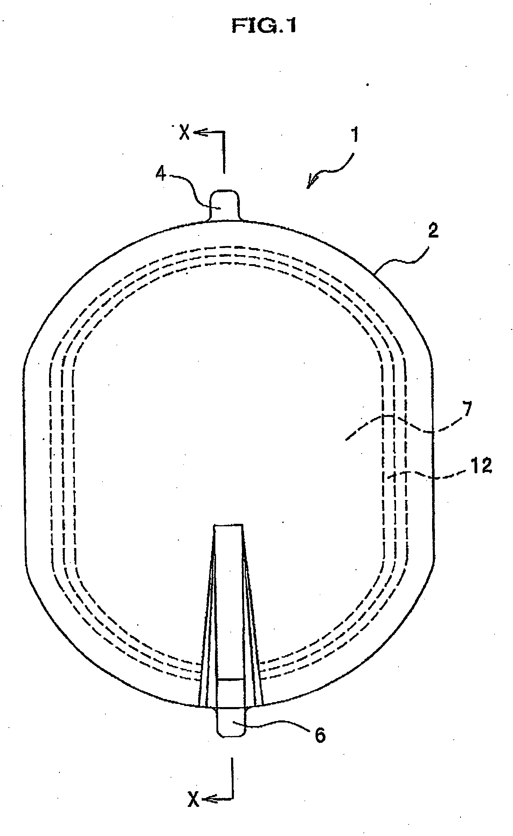

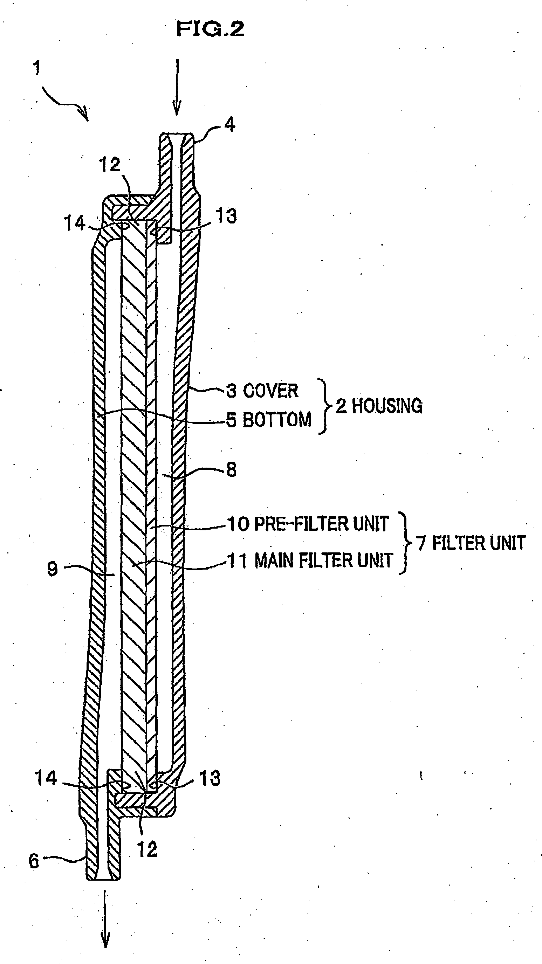

FIG. 1 is a plan view of the blood treatment filter. FIG. 2 is an X-X cross sectional view of FIG. 1.

<Blood Treatment Filter>

As shown in FIGS. 1 and 2, a blood treatment filter 1 includes: a housing 2; a filter unit 2 stored in the housing 2 for filtering blood which contains erythrocytes, platelets, and leukocytes as blood components. The respective units will be explained below. Here, blood used in the embodiment is preferably blood within 24 hours after collected from a donor. If blood is kept for more than 24 hours after blood collection from a donor, aggregates are formed in the blood by aggregation of proteins and blood components. Therefore, when the blood is filtered, the aggregates are captured in the filter unit 7 (pre-filer 10) and block the filter, resulting in blood not easily flowing.

(Housing)

The housing 2 includes: a disk-like cover 3 having one end at which an inlet 4 is formed for introducing blood; and a disk-like bottom 5 having one end at which an outlet 6 is formed for draining the blood treated by the filter unit 7.

The cover 3 and the bottom 5 are joined together at the respective peripherals to form an inner space for holding the filter unit 7. The filter unit is stored in the inner space and a periphery of the filter unit 7 is sandwiched between the cover 3 and the bottom 5. Hereon, the contact portions are fusion-bonded by high frequency fusion or ultrasonic fusion. By the procedure, the respective portions of the cover 3, the filter 7 and the bottom 5 are connected in a liquid-tight state. Then, the inner space is divided by the filter unit 7 into a blood inlet space 8 communicating with the inlet 4 and a blood outlet space 9 communicating with the outlet 6.

For the cover 3 and the bottom 5, it is preferable that step portions 14, 13 for holding the filter unit are provided at the respective peripheries to hold the filter unit 7. By providing the step portions 14, 13, the filter unit 7 is held steadfast between the cover 3 and the bottom 5. Hereby, blood introduced into the blood inlet space 8 necessarily passes through the filter 7 to flow into the blood outlet space 9.

The cover 3 and the bottom 5 are made of a resin molding member. The preferable component material is: polycarbonate, polyvinyl chloride, flexible polyvinyl chloride, ethylene-vinyl acetate copolymer, and acrylnitrile-butadiene-styrene copolymer (ABS resin). Further, the cover 3 and the bottom 5 may be made of different resin or the same resin.

(Filter Unit)

The filter unit 7 includes a main filter unit 11 and a pre-filter unit 10 which is arranged at the up-stream side of the main filter unit 11.

The filter unit 7 is prepared by fusion-bonding peripheries of the main filter 11 and the pre-filter 10 through high frequency fusion or ultrasonic fusion. By the fusion-bonding process, communication holes for filtering blood are closed and non-filtering function unit (fusion-bonded unit 12) is formed. Since in the non-filtering function unit (fusion-bonded unit 12), blood tends to stay leading to a decrease of a treated blood amount, it is preferable that the non-filtering function unit (fusion-bonded unit 12) is not exposed to the blood inlet space 8 and the blood outlet space 9. Here; formation of the fusion-bonded unit 12 may be formed before storing the unit in the housing 2, or formed at the same time as the fusion-bonding with the housing 2 (cover 3 and bottom 5).

It is preferable that a treated blood amount per unit volume for the filter unit 7 is 10 to 20 mL/cm$^3$, when blood is passed by gravity. If the treated blood amount is under 10 mL/cm$^3$, a recovery rate of other blood components other than predetermined blood components such as platelets and/or erythrocytes, tends to be decreased. In contrast, if the treated blood amount is over 20 mL/cm$^3$, a removal rate (filtering rate) of the predetermined blood components such as leukocytes, tends to be decreased.

Further, it is preferable that a thickness of the filter unit is 4 to 20 mm, more preferably 4 to 10 mm. If the thickness is under 4 mm, a treated blood amount per unit volume tends to be smaller. If the thickness is over 20 mm, the treated blood amount per unit volume tends to be larger. Further, it is preferable that a volume of the filter unit 7 is 4 to 10% of the treated blood amount (blood collecting amount from a donor). If the volume is under 4%, the treated blood amount per unit volume tends to be smaller. If the volume is over 10%, the treated blood amount per unit volume tends to be larger.

Furthermore, it is preferable that a volume ratio between the pre-filter unit 10 and the main filter unit 11 is 1:5 to 1:20, more preferably 1:7 to 1:15. If the volume ratio is under 1:5, that is, if a volume of the main filter unit 11 is small, a removal rate (filtering rate) of the predetermined blood cell components such as leukocytes tends to be decreased. On the contrary, if the volume ratio is over 1.20, a recovery rate of blood cell components besides predetermined blood cell components such as platelets and/or erythrocytes tends to be decreased.

Next, the main filter unit 11 and pre-filter unit 10 which constitute the filter unit 7 will be further explained.

(Main Filter Unit)

The main filter unit 11 virtually removes predetermined blood cell components from blood and passes the remaining blood cell components. Therefore, it is preferable that the main filter unit 11 is made of a sponge-like porous membrane having a number of micro communication holes passing one side of the main filter 11 to the other side and having a liquid permeable structure, or of nonwoven fabric. With respect to component material for the main filter 11, if a sponge-like porous membrane is used, polyether type polyurethane and polyester type polyurethane are preferable. If nonwoven fabric is utilized, polyethylene terephthalate and polybutylene terephthalate are preferable.

With respect to the main filter 11, the absolute surface zeta potential value is not particularly restricted. If the filter substantially removes only leukocytes from blood and passes platelets and erythrocytes, the absolute surface zeta potential value is -2.0 to +2.0 mV, preferably +0.5 to 1.0 mV. If the absolute surface zeta potential value is under -2.0 mV or over +2.0 mV, the other blood cell components besides leukocytes are removed and the recovery rate of the blood cell components (platelets and erythrocytes) tends to be decreased. On the other hand, if the main filter 11 removes leukocytes and platelets from blood and passes erythrocytes through the filter, in case of nonwoven fabric, it is preferable that the absolute surface zeta potential value is -20 to -30 mV, or in case of a sponge-like porous membrane, it is preferable that the absolute surface zeta potential value is +16 to +18 mV.

With respect to a method for having the absolute surface zeta potential value of the main filter 11 within predetermined range, there are a method for preparing the main filter unit 11 by component material having a predetermined absolute surface zeta potential value, or a method for introducing material having a functional group with electric potential, for example, a cationic functional group like an amino group into a surface of the main filter unit 11, by using chemical reactions.

For the main filter unit 11, it is preferable that when a suspension containing first micro particles having an average particle size of 3 $\mu$m, a removal rate of the first micro particles is 85 to 99%. If the removal rate is under 85%, the removal rate (filtering rate) of the predetermined blood components such as leukocytes tends to be decreased. Alternatively, if the removal rate is over 99%, the recovery rate of the other blood cell components other than the predetermined blood cell components such as platelets and/or erythrocytes tends to be decreased.

. The main filter unit 11 substantially removes only leukocytes as a predetermined blood cell component from blood, and passes erythrocytes and platelets as the other blood cell components other than the predetermined blood components through the filter. Herein, the removal rate of the predetermined blood cell component (leukocytes) is improved, and the recovery rate of the other blood cell components (erythrocytes and platelets) other than the predetermined blood cell components is mostly increased.

(Pre-filter Unit)

With respect to the pre-filter unit 10, it is preferable that the pre-filter does not substantially remove (namely passes) blood cell components from blood, and is made of a sponge-like porous membrane having a number of micro communication holes communicating one side with the other side and having a structure of liquid permeability. With respect to component material for the pre-filter unit 10, polyether type polyurethane and polyester type polyurethane are preferable. The material may be made of different resin from the main filter unit 11, or made of the same resin.

With respect to the pre-filter unit 10,
an absolute surface zeta potential value is -2.0 to +2.0 mV,
preferably +0.5 to +1.0 mV. If the absolute surface zeta potential value is under -2.0 mV or over +2.0 mV, blood cell components are captured in the pre-filter unit 10 and a recovery rate of blood cell components such as platelets and/or erythrocytes is decreased. Herein, a method for giving the preferable absolute surface zeta potential value is the same as conducted for the main filter unit 11.

With respect to the pre-filter unit 10, when a suspension containing second micro particles having an average particle size of 6 $\mu$m is passed by gravity, a permeability rate of the second micro particles needs to be 70 to 90%. If the permeability rate is out of the predetermined range, a recovery rate of the other blood cell components other than the predetermined blood cell components such as platelets and/or erythrocytes is decreased.

<Blood Treatment Circuit>

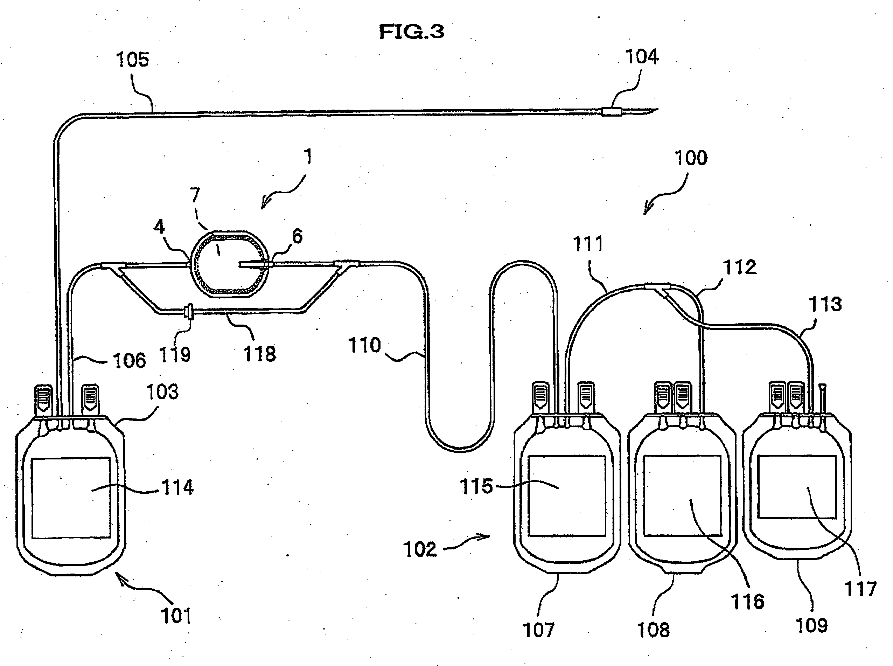

Next, the blood treatment circuit of the present invention will be explained in detail with referring to drawings. FIG. 3 is a plan view of the blood treatment circuit.

As shown in FIG. 3, a blood treatment circuit 100 comprises: the blood treatment filter 1, a blood collecting apparatus 101 which communicates to the inlet of the blood treatment filter 1, and a blood treatment apparatus 102 which communicates to the outlet 6 of the blood filter unit 1. Hereafter, the respective components will be explained. Here, the blood filter 1 will not be explained because it is already explained in the previous description.

(Blood Collecting Apparatus)

The blood collecting apparatus 101 includes a primary bag 103 for storing collected blood. Further, at an upper end portion of the primary bag 103, are connected a second tube 106 having flexibility to communicate to the inlet 4, and a first tube 105 having flexibility to communicate to a blood collecting needle 104 for collecting blood from a donor. Here, a sampling line may be connected by setting a three-way branching connector along the first tube 105, not shown.

The primary bag 103 is made of laminated flexible sheets and prepared by fusion-bonding (sealing) the periphery of the sheet to form a bag-like shape. However, if the bag is capable of storing the collected blood, the shape is not limited in particular. For example, a bag may be a tubular sheet, with its open ends fusion-bonded (sealed), or from a folded sheet, with the three open sides fusion-bonded (sealed).

It is preferable that the primary bag 103 contains an anticoagulant in advance. The anticoagulant is preferably a liquid such as ACD-A solution, CPD solution, CPDA-1 solution, and heparin sodium solution. The amount in the bag varies depending on the amount of blood to be collected. Further, outside the primary bag 103, a label 114 can be attached on which is described information on bag contents such as a kind of blood components stored (blood cell components), a capacity of the bag, a blood group, blood collection date, donor information.

For component material of the primary bag 103 and the first and second tubes 105 and 106, such the material as selected from polyvinyl chloride, flexible polyvinyl chloride, material mainly containing flexible polyvinyl chloride (and its copolymer, polymer blended, or polymer alloy with a small amount of other polymers), ethylene-vinyl acetate copolymer, is preferable.

(Blood Treatment Apparatus)

The blood treatment apparatus 102 includes the secondary bag 107 for collecting remaining blood cell components which flow out from the blood treatment filter 1 (outlet 6) and from which the predetermined blood cell components are removed. At the upper end of the secondary bag 107, a flexible third tube 110 which communicates to the outlet 6 is connected.

It is preferable that in the blood treatment apparatus 102, in addition to the secondary bag 107, a third bag 108 and a forth bag 109 for storing a blood cell component which is separated into a respective component are connected with the secondary bag 107 in parallel through flexible forth, fifth, and sixth tubes 111, 112, and 113. For example, after centrifugal separation of blood cell components from which leukocytes are removed by the blood filter 1, the resulting separated concentrated red blood cell liquid is stored in the secondary bag 107, the separated concentrated platelets are stored in the third bag 108, and the separated platelet-poor plasma is stored in the forth bag 109. Here, the forth, fifth and sixth tubes 111, 112, and 113 are connected through a three-branching connector (T-tube, Y-tube, branch-tube, three-way cock). Similarly to the primary bag 103, a label 115, 116, and 117 for describing storage information may be attached on the outside of the secondary, third, and forth bags 107, 108 and 109, respectively.

The secondary, third and forth bags 107, 108, and 109 are formed of laminated flexible sheets, with their periphery heat-sealed. The bags may be a tubular sheet, with its open ends fusion-bonded (sealed), or from a folded sheet, with the three open sides fusion-bonded (sealed). They are not specifically restricted in their shape.

The forth bag 109 contains a medical liquid in advance. The medical liquid is preferably an erythrocyte storage solution such as SAGM solution, OPTISOL solution, and MAP solution. The amount in the bag varies depending on the amount of blood to be collected (treated blood amount).

A component material for the secondary bag 107, the third bag 108, and the forth bag 109 is selected from any of the component materials exemplified in the primary bag 103. The material may be made of a different resin from the resin used for the primary bag 103, or made of the same resin as the primary bag 103. The component material for the third, forth, fifth, and sixth tubes 110, 111, 112, and 113 is selected from any of the material exemplified in the first and the second tubes 105 and 106, and is preferably made of the same resin as the first and the second tubes 105 and 106.

According to another embodiment of the blood treatment circuit 100, a by-pass tube 118 which goes around the blood treatment filter 1 and an air vent (not shown) may be provided between the second tube 106 in the blood collecting apparatus 101 and the third tube 110 in the blood treatment apparatus 102. By providing the by-path tube and the air vent, air which came in the bag 107 is easily discharged. A stopper 119 is provided with the by-path tube 118. The stopper 19 prevents blood in the primary bag 103 from flowing into the secondary bag 107 through the by-path tube 118 without passing through the blood treatment filter 1. Furthermore, air came in the secondary bag 107 is possibly discharged to the primary bag 103 through the by-path tube 118.

Further, by providing the air vent, since the air vent admits gas or heat into the blood treatment filter 1 at the time of sterilization, rapid sterilization of the filter unit 7 in the blood treatment filter 1 is ensured. Herein, the blood treatment filter 1 may be connected with the second tube 106 and the third tube 110 by using a sterile tubing welder disclosed in Japanese Laid-Open Patent Publication Nos. H9-154920 and 2002-308688.

Next, a method for using the blood treatment circuit of the present invention will be explained. Herein, a predetermined blood cell component is leukocytes and the blood treatment filter 1 is a leukocyte removing filter. Further, the blood treatment apparatus 102 includes the third and forth bags 108 and 109 in addition to the secondary bag 107.

(1) Blood is collected from a donor (blood supplier) by puncturing the blood collecting needle 104 of the blood collecting apparatus 101 into the donor's vessel. Collected blood is stored in the primary bag 103 through the first tube 105. Here, it is preferable that the second tube 106 is sealed with a breakable stopper to prevent blood from flowing from the primary bag 103 to the second tube 106.

(2) The seal of the second tube 106 with the breakable stopper is released, and the blood in the primary bag 103 is discharged through the second tube 106. The discharged blood flows into the blood treatment filter 1 from the inlet 4 through the second tube 106. In this case, the primary bag 103 holding blood is suspended at a high position from a stand so that blood flows by gravity.

(3) The introduced blood is filtered and leukocytes are removed in the filter unit 7. The blood from which leukocytes are removed (leukocyte-free blood) is discharged from the outlet 6 and introduced into the second bag 107 of the blood treatment apparatus 102 through the third tube 110.

(4) After the leukocyte-free blood has been collected in the secondary bag 107, the tube 110 is sealed by fusion-bonding at a position near the secondary bag 107 by using a tube sealer. Then the sealed part is cut to separate the blood collecting apparatus 101 and the blood treatment filter 1 from the blood treatment apparatus 102.

(5) All of the secondary bag 107, the third bag 108 and the forth bag 109 of the blood collecting apparatus 101 are put together in the cup of the centrifuge and the leukocyte-free-blood is centrifuged. As a result, for example, the leukocyte-free blood in the secondary bag 107 is separated into two layers; the lower layer containing erythrocytes and the upper layer containing platelet rich plasma. The pattern of separation of blood components depends on the conditions of centrifugation (such as speed and duration time of rotation).

(6) Then, the upper layer containing platelet rich plasma in the secondary bag 107 is transferred to the third bag 108 through, the forth and fifth tubes 111 and 112, via a pressuring operation by a blood component separation device (not shown) - At this time, the sixth tube 113 is sealed with a clamp. As the result of the above-mentioned procedure, erythrocytes remain in the secondary bag 107.

(7) After transfer of platelet rich plasma is completed, the clamp on the sixth tube 113 is released. With the fifth tube 112 sealed with a clamp, the erythrocyte storage solution in the forth bag 109 is transferred to the secondary bag 107 through the sixth and the forth tubes 113 and 111. A portion in.the forth tube 111 is sealed by fusion-bonding with a tube sealer and the sealed part is cut to separate the secondary bag 107 from the third and the forth bags 108 and 109. Hereby, concentrated red blood cells are recovered in the secondary bag 107.

(8) The third bag 108 and the forth bag 109 are put together in the cup of the centrifuge for centrifugation. As a result, the platelet rich plasma in the third bag 108 is separated into platelet pellet (precipitate) and supernatant plasma (platelet-poor plasma). The pattern of separation of blood components depends on the conditions of centrifugation.

(9) The third bag 108 is set on a blood component separation device, and the supernatant plasma is discharged and transferred by pressing to the forth bag 109 through the fifth and the sixth tubes 112 and 113. Portions in the fifth and the sixth tubes 112 and 113 are sealed by fusion-bonding with a tube sealer, and the sealed parts are cut to separate the third bag 108 and the forth bag 109 from each other. Hereby, concentrated platelets are collected in the third bag 108 and platelet-poor plasma is collected in the forth bag 109, respectively.

EXAMPLES

The following is the detailed description about the embodiments which cover the blood treatment filter of the present invention.

(Examples 1 to 17)

A blood treatment filter including a housing and a filter unit is assembled through the following steps. With respect to components, the housing includes a cover and a bottom which are made of polycarbonate with the shape shown in FIGS. 1 and 2. The filter unit includes a main filter unit made of a sponge-like porous membrane of polyether type polyurethane, and a pre-filter unit made of a sponge-like porous membrane of polyether type polyurethane. Firstly, the periphery of the filter unit assembled with the main filter and the pre-filter units is fusion-bonded by high frequency fusion-bonding, and the filter unit is held in the housing. Then, the periphery of the housing is fusion-bonded by high frequency fusion-bonding from the outside to prepare the blood treatment filter. Here, the effective area of the filter unit excluding the fusion-bonding part (non-filtering functional position) is 50 cm$^2$.

(Example 18) (useful for understanding the invention)

A filter unit including a main filter unit and a pre-filter unit is held in a housing which includes a cover and a bottom. Both cover and the bottom are made of polycarbonate with the shape shown in FIGS. 1 and 2. The main filter unit is made of nonwoven fabric of polyethylene terephthalate having an average fiber diameter of 1.2 $\mu$m, and the pre-filter unit is made of a sponge-like porous membrane of polyether type polyurethane. The filter unit is fusion-bonded at the respective peripheries by high frequency fusion-bonding and is held in the housing. Then, the periphery of the housing including the filter unit is fusion-bonded by high frequency fusion-bonding to assemble the blood treatment filter. Herein, the effective area excluding the fusion-bonding part (non-filtering functional position) of the filter unit is 50 cm$^2$.

Here, the surface of the main filter unit is covered with copolymer made of 2-hydroxyethyl methacrylate (HEMA), dimethylaminoethyl methacrylate (DM).

(Comparative Example 1)

A blood treatment filter is prepared by the same method as examples 1 to 17 except for a filter unit. Herein, the filter unit includes only a pre-filter unit.

(Comparative Example 2)

A blood treatment filter is prepared by the same method as examples 1 to 17 except for a filter unit. Herein, the filter unit includes only a main filter unit.

(Comparative Example 3)

A blood treatment filter is prepared by the same method as examples 1 to 17 except for a pre-filter unit. Herein, the pre-filter unit is made of a sponge-like porous membrane of polyether type polyurethane of which permeability rate of micro particles (6 $\mu$m) is under the smallest value in the preferable range of the present invention.

(Comparative Example 4)

A blood treatment filter is prepared by the same method as examples 1 to 17 except for a pre-filter unit. Herein, the pre-filter unit is made of a sponge-like porous membrane of polyether type polyurethane of which permeability rate of micro particles (6 $\mu$m) is over the largest value in the preferable range of the present invention.

(Comparative Example 5)

A blood treatment filter is prepared by the same method as example 18 except for a pre-filter unit. Herein, the pre-filter unit is made of nonwoven fabric of polyethylene terephthalate having an average fiber diameter of 32 $\mu$m instead of the sponge-like porous membrane of polyether type polyurethane.
Here, a surface of the pre-filter unit is covered with copolymer made of N, N-bis(hydroxyethyl)amino-2-hydroxypropyl methacrylate (GA) and 2-hydroxyethyl methacrylate (HEMA).

(Comparative Example 6)

A blood treatment filter is prepared by the same method as example 18 except for a filter unit. Herein, the filter unit includes only a main filter unit.
Here, a permeability rate or a removal rate of micro particles, a volume, a volume ratio, and an absolute surface zeta potential value of the main filter unit and the pre-filter unit are listed in Table 1 and Table 2. The permeability or removal rate of micro particles and the absolute surface zeta potential value are measured in the following method.

(Permeability or Removal Rate of Micro Particles)

(1) A sponge-like porous membrane of polyether type polyurethane for the main filter unit and nonwoven fabric of polyethylene terephthalate (the surface is covered with copolymer) for the pre-filter unit are blanked so that each effective area is 50 cm$^3$. The blanked membrane and fabric are set in a special holder for this comparison experiment.
(2) As an evaluating solution, a solution of 50 mL is prepared by dispersing mono disperse micro particles MX (Soken Chemical Engineering Co., Ltd.) in a 0.5 w/v % SDS (sodium lauryl sulfonate) solution to be suspended in a predetermined concentration. Herein, the property of MX is as follows: material- particulate cross linked acrylic polymer (PMMA); specific gravity- 1.10; refractive index- 1.49; CV value (variable coefficient-ratio of standard deviation to average particle size) is about 9.0. Further, for evaluating the pre-filter unit, is used a solution prepared by dispersing MX-600 (average particle size 6.0 $\mu$m) as micro particles to be suspended in a concentration of 200 ppm (w/w). For evaluating the main filter unit, is used a solution prepared by dispersing MX-300 (average particle size 3.0 $\mu$m) as micro particles to be suspended in a concentration of 100 $\mu$m (w/w).
(3) The evaluating solution is passed through the porous membrane and nonwoven fabric by gravity (natural fall by self-weight) so that an average flow speed is 50 mL/min to filtrate the micro particles.
(4) After filtration, the micro particles collected are analyzed by a counter analyzer (RS Co., Ltd., HIAC Royoco, automatic liquid micro particle analyzer) to calculate the permeability and removal rate, respectively.

(Absolute Surface Zeta Potential Value)

A flowing liquid is passed through the porous membrane and the nonwoven fabric. Hereby, a streaming potential value, a pressure added to pass the flowing liquid, and conductivity of the flowing liquid are measure by streaming potential measuring device (Shimazu, ZP20H). The absolute surface zeta potential value is calculated according to the following equation (1). For the measurement, KC1 aqueous solution (1 mM) is used for the flowing liquid. The above-mentioned data is measured at pH 6 + 1 and temperature 20 + 5°C.

$$\text{Absolute surface zeta potential value (mV)}$$

$$= (4\pi\eta / \varepsilon) \times (Es\lambda / P) \; --- \; (1)$$

In, the equation, $\eta$ indicates viscosity of the flowing liquid, $\varepsilon$ indicates impedance of the flowing liquid, Es indicates flowing potential, $\lambda$ indicates an electric conductivity, and P indicates a pressure put for transferring the flowing liquid.

Next, by using the blood treatment filters in examples 1 to 18 and comparative examples 1 to 6, blood as to correspond to about 570 mL (blood within 24 hours after blood collection from a donor) is filtered. Each of the treated blood amounts (mL/cm$^3$) at the filtration is listed in Table 1. The number of leukocytes before and after the filtration is measured by automated hematology analyzer (Sysmex Co., Ltd., XE-2100) and flow cytometer FCM (Beckman-Coulter Co., Ltd., EPICS-XL, LeukoSure Kit) to calculate a leukocyte removal rate, platelet recovery rate, erythrocyte recovery rate. Additionally, the time required for filtering blood as to correspond to about 570 mL (filtration duration time) is measured. The resulting data are shown in Table 1. Here, the blood filtration performance (leukocyte removal rate, platelet recovery rate, erythrocyte recovery rate, and filtration duration time) is evaluated based on the following standards.

(Leukocyte Removal Rate)

Excellent: leukocyte removal rate > 3.8 (-Log); Good: 3.8 (-Log) > leukocyte removal rate > 3.0 (-Log); Poor: 3.0 > leukocyte removal rate

(Platelet Recovery Rate)

Excellent: platelet recovery rate > 75%; Good: 75% > platelet recovery rate > 60%; 60% > platelet recovery rate

(Erythrocyte Recovery Rate)

Excellent: erythrocyte recovery rate > 90%; Good: 90% > erythrocyte recovery rate > 85%; Poor: 85% > erythrocyte recovery rate

(Filtration Duration Time)

Excellent: 45 min > filtration duration time; Good: 60 min > filtration duration time > 45 min; Poor: filtration duration time > 60 min

Table 1

| | filter unit | | | | | blood filtration performance | | | | |
|---|---|---|---|---|---|---|---|---|---|---|
| | pre-filter unit (A) | main filter unit (B) | volume (A) + (B) (cm³) | treated blood amount (mL/cm³) | volume ratio (A):(B) | leukocyte removal rate (-Log) | platelet recovery rate (%) | erythrocyte recovery rate (%) | filtration duration time | total performance |
| | micro particle (6μm) pearmeability rate (%) | micro particle (3 μm) removal rate (%) | | | | | | | | |
| example 1 | 80 | 85 | 48 | 12 | 1:7 | 4.1 | 82 | 92 | E | E |
| example 2 | 80 | 99 | 48 | 12 | 1:7 | 4.7 | 75 | 91 | E | E |
| example 3 | 70 | 95 | 48 | 12 | 1:7 | 4.1 | 77 | 91 | E | E |
| example 4 | 90 | 95 | 48 | 12 | 1:7 | 4.1 | 79 | 91 | E | E |
| example 5 | 80 | 95 | 48 | 12 | 1:7 | 4.5 | 81 | 93 | E | E |
| example 6 | 80 | 95 | 25 | 20 | 1:7 | 4.1 | 80 | 95 | E | E |
| example 7 | 80 | 95 | 50 | 10 | 1:7 | 4.5 | 75 | 90 | E | E |
| example 8 | 80 | 95 | 48 | 12 | 1:5 | 3.8 | 85 | 92 | E | E |
| example 9 | 80 | 95 | 48 | 12 | 1:7 | 4.3 | 81 | 92 | E | E |
| example 10 | 80 | 95 | 48 | 12 | 1:15 | 4.3 | 80 | 92 | E | E |
| example 11 | 80 | 95 | 48 | 12 | 1:20 | 4.5 | 79 | 91 | E | E |
| example 12* | 80 | 50 | 48 | 12 | 1:7 | 3.3 | 84 | 93 | E | G |
| example 13* | 80 | 99.5 | 48 | 12 | 1:7 | 4.9 | 60 | 88 | G | G |
| example 14* | 80 | 95 | 20 | 25 | 1:7 | 3.0 | 89 | 99 | E | G |
| example 15* | 80 | 95 | 75 | 6.7 | 1:7 | 4.8 | 63 | 85 | E | G |
| example 16 | 80 | 95 | 48 | 12 | 1:3 | 3.1 | 85 | 92 | E | G |
| example 17 | 80 | 95 | 48 | 12 | 1:30 | 4.9 | 60 | 89 | G | G |
| example 18* | 80 | 99 | 48 | 12 | 1:7 | 4.1 | 82 | 91 | E | G |
| comparative example 1 | 80 | - | 6 | 95 | - | 0.01 | 97 | 99 | E | P |

EP 2 055 329 B1

(continued)

| | filter unit | | | | | blood filtration performance | | | | |
|---|---|---|---|---|---|---|---|---|---|---|
| | pre-filter unit (A) | main filter unit (B) | volume (A) + (B) (cm³) | treated blood amount (mL/cm³) | volume ratio (A):(B) | leukocyte removal rate (-Log) | platelet recovery rate (%) | erythrocyte recovery rate (%) | filtration duration time | total performance |
| | micro particle (6μm) pearmeability rate (%) | micro particle (3 μm) removal rate (%) | | | | | | | | |
| comparative example 2 | - | 95 | 42 | 13.5 | - | 4.5 | 29 | 49 | P | P |
| comparative example 3 | 50 | 95 | 48 | 12 | 1:7 | 4.2 | 61 | 90 | P | P |
| comparative example 4 | 93 | 95 | 48 | 12 | 1:7 | 4.5 | 60 | 88 | P | P |
| comparative example 5 | 95 | 99 | 46 | 128 | 1:14 | 4.1 | 50 | 92 | G | P |
| comparative example 6 | - | 99 | 42 | 13.5 | - | 4.6 | 35 | 63 | G | P |
| E: Excellent G:Good P:Poor * useful for understanding the invention | | | | | | | | | | |

EP 2 055 329 B1

Table 2

| | absolute surface zeta potential value of filter unit | |
|---|---|---|
| | pre-filter unit | main filter unit |
| example 1 | +0.8 | +0.8 |
| example 2 | +0.8 | +0.8 |
| example 3 | +0.8 | +0.8 |
| example 4 | +0.8 | +0.8 |
| example 5 | +0.8 | +0.8 |
| example 6 | +0.8 | +0.8 |
| example 7 | +0.8 | +0.8 |
| example 8 | +0.8 | +0.8 |
| example 9 | +0.8 | +0.8 |
| example 10 | +0.8 | +0.8 |
| example 11 | +0.8 | +0.8 |
| example 12* | +0.8 | +0.8 |
| example 13* | +0.8 | +0.8 |
| example 14* | +0.8 | +0.8 |
| example 15* | +0.8 | +0.8 |
| example 16 | +0.8 | +0.8 |
| example 17 | +0.8 | +0.8 |
| example 18* | +0.8 | -25 |
| comparative example 1 | +0.8 | - |
| comparative example 2 | - | +0.8 |
| comparative example 3 | +0.8 | +0.8 |
| comparative example 4 | +0.8 | +0.8 |
| comparative example 5 | -21 | -25 |
| comparative example 6 | - | -25 |
| * useful for understanding the invention | | |

According to the results in Table 1 and Table 2, the performance of the blood treatment filters in examples 1 to 11 is excellent on all of the evaluation standards: leukocyte removal rate, platelet recovery rate, erythrocyte recovery rate, and filtration duration time. Therefore, it is confirmed that the blood treatment filters have excellent total performance in all of the blood filtering standards.

The blood treatment filters in examples 12 to 18 show good performance, although at least one of leukocyte removal rate, platelet recovery rate, erythrocyte recovery rate and filtration duration time in the above examples is lower than the evaluation in examples 1 to 11. The lower evaluation is attributed to that one value among the micro particle removal rate, absolute surface zeta potential value of the main filter unit, treated blood amount, or volume ratio of the filter unit is out of the preferable range of the present invention.

With respect to the blood treatment filter in comparative example 1, the leukocyte removal rate is poor due to lack of the main filter unit. With respect to the blood treatment filter in comparative examples 2 and 6, the platelet recovery rate and erythrocyte recovery rate are poor due to lack of the pre-filter unit. With respect to the blood treatment filter in comparative example 3, the movement of the blood cells introduced into the main filter unit is not sufficiently controlled because the blood cell components (mainly leukocytes) are removed in the pre-filter unit due to the low micro particle permeability rate in the pre-fliter unit. With respect to the blood treatment filter in comparative example 4, the movement of the blood cells introduced into the main filter unit is not sufficiently controlled due to the low micro particle rate of the pre-filter unit. As the result, the filtration duration time of the blood treatment filters in comparative examples 3 and 4 is evaluated as poor. Further, with respect to the blood treatment filter in comparative example 5, the platelet recovery rate is evaluated as poor because blood cell components (leukocytes and platelets) are adsorbed in the pre-filter unit due to too low absolute surface zeta potential value of the pre-filter unit. Thus, with respect to the blood treatment filters in comparative examples 1 to 6, it is confirmed that their total judgement of performance is poor.

**Claims**

1. A blood treatment filter (1) including,
   a housing (2) having an inlet (4) and an outlet (6), and
   a filter unit (7) which is stored in the housing (2) and filters blood containing erythrocytes, platelets, and leukocytes as blood components, wherein
   the filter unit (7) includes a main filter unit (11) for substantially removing leukocytes from the blood and passing the other blood components there through, and a pre-filter unit (10) which passes the blood components there through, and
   the main filter unit (11) has a removal rate of 85 to 99% for first micro particles having an average particle size of 3 $\mu$m which are contained in a suspension passing there through by gravity so that an average flow speed of the suspension is 50 mL/min, and the absolute surface zeta potential value of the main filter (11) is -2.0 to +2.0 mV
   the pre-filter unit (10) has a permeability rate of 70 to 90% for micro particles having an average particle size of 6 $\mu$m which are contained in the suspension passing there through by gravity so that an average flow speed of the suspension is 50 mL/min, and the absolute surface zeta potential value of the pre-filter (10) is -2.0 to +2.0 mV, and
   the filter unit (7) has a treated blood amount per unit volume of 10 to 20 mL/cm$^3$ for the blood which passes there through by gravity.

2. The blood treatment filter according to claim 1, wherein
   a volume ratio between the pre-filer unit (10) and the main filter unit (11) is 1:5 to 1:20.

3. The blood treatment filter (1) according to claim 1, wherein
   the main filter unit (11) and the pre-filter unit (10) are sponge-like porous membranes having a number of micro communication holes communicating with one side of the membrane and the other side, and a structure of fluid permeability.

4. The blood treatment filter (1) according to claim 1, wherein
   the blood is treated within 24 hours after blood collection from a donor.

5. The blood treatment filter (1) according to claim 1, wherein
   an absolute surface zeta potential value of the main filter unit (11) is +0.5 to +1.0 mV, and an absolute surface zeta potential value of the pre-filter unit (10) is +0.5 to +1.0 mV.

6. A blood treatment circuit (100) comprising:

   the blood treatment filter (1) according to claim 1;
   a blood collecting apparatus (101) which communicates with the inlet (4) of the blood treatment filter (1) and includes a primary bag (103) for storing the collected blood; and
   a blood treatment apparatus (102) which communicates with the outlet (6) of the blood treatment filter (1) includes a secondary bag (107) for collecting the remaining blood components which flow out from the outlet (6) and which are produced by removing the predetermined blood components.

7. The blood treatment circuit (100) according to claim 6, wherein the blood collecting apparatus (101) comprises:

   the primary bag (103);
   a blood collecting needle (104);
   a first tube (105) including an end connected to the primary bag (103) and the other end connected to the blood collecting needle (104); and
   a second tube (106) including an end connected to the primary bag (103) and the other end connected to the inlet (4)of the blood treatment filter (1).

8. The blood treatment circuit according to claim 6, wherein the blood treatment apparatus (102) comprises:

the secondary bag (107);
a third tube (110) including an end connected to the outlet (6) of the blood treatment filter (1) and the other end connected to the secondary bag (107);
a third bag (108) for storing the blood cell components separated from the blood cell components stored in the secondary bag (107);
a forth bag (109) in which a medical liquid is filled beforehand;
a forth tube (111) including anend connected to the secondary bag (107);
a fifth tube (112) including an end connected to the forth tube (111) and the other end connected to the third bag (108); and
a sixth tube (113) including anend connected to the forth tube (111) and the other end connected to the forth bag (109).

## Patentansprüche

1. Blutbehandlungsfilter (1) beinhaltend,
ein Gehäuse (2) mit einem Einlass (4) und einem Auslass (6), und
eine Filtereinheit (7), welche in dem Gehäuse (2) aufbewahrt ist, und das Blut filtriert, welches Erythrozyten, Plättchen und Leukozyten als Blutbestandteile enthält, wobei
die Filtereinheit (7) eine Hauptfiltereinheit (11), für die substantielle Entfernung von Leukozyten aus dem Blut und die andere Blutbestandteile dadurch passieren lässt, und eine Vorfiltereinheit (10) beinhaltet, welche die Blutbestandteile dadurch passieren lässt, und
die Hauptfiltereinheit (11) eine Entfernungsrate von 85 bis 99% für erste Mikropartikel mit einer durchschnittlichen Partikelgröße von 3 $\mu$m aufweist, welche in einer Suspension enthalten sind, die dadurch mittels Gravitation passieren, sodass eine durchschnittliche Fließgeschwindigkeit der Suspension 50 mL/min ist, und der absolute Wert des Zeta-Potentials der Oberfläche des Hauptfilters (11) -2,0 bis +2,0 mV ist,
die Vorfiltereinheit (10) eine Durchlässigkeitsrate von 70 bis 90% für Mikropartikel mit einer durchschnittlichen Partikelgröße von 6 $\mu$m aufweist, die in der Suspension enthalten sind, die dadurch mittels Gravitation passieren, sodass eine durchschnittliche Fließgeschwindigkeit der Suspension 50 mL/min ist, und der absolute Wert des Zeta-Potentials der Oberfläche des Vorfilters (10) -2,0 bis +2,0 mV ist, und
die Filtereinheit (7) eine Blutbearbeitungsmenge pro Volumeneinheit von 10 bis 20 mL/cm$^3$ für das Blut aufweist, welches dadurch mittels Gravitation passiert.

2. Blutbehandlungsfilter nach Anspruch 1, wobei
ein Volumenverhältnis zwischen der Vorfiltereinheit (10) und der Hauptfiltereinheit (11) 1:5 bis 1:20 ist.

3. Blutbehandlungsfilter (1) nach Anspruch 1, wobei
die Hauptfiltereinheit (11) und die Vorfiltereinheit (10) schwammartige poröse Membranen sind, die eine Anzahl von Mikroverbindungslöchern, die mit der einen Seite der Membran und der anderen Seite in Verbindung stehen, und eine Struktur der Flüssigkeitsdurchlässigkeit aufweisen.

4. Blutbehandlungsfilter (1) nach Anspruch 1, wobei
das Blut innerhalb von 24 Stunden nach Blutentnahme vom Spender behandelt wird.

5. Blutbehandlungsfilter (1) nach Anspruch 1, wobei
ein absoluter Wert des Zeta-Potentials der Oberfläche der Hauptfiltereinheit (11) +0,5 bis +1,0 mV ist, und absoluter Wert des Zeta-Potentials der Oberfläche der Vorfiltereinheit (10) +0,5 bis +1,0 mV ist.

6. Blutbehandlungskreislauf (100) umfassend:

den Blutbehandlungsfilter (1) nach Anspruch 1;
einen Blutaufnahmegerät (101), welches mit dem Einlass (4) des Blutbehandlungsfilter (1) in Verbindung steht und einen primären Beutel (103) für das Aufbewahren des gesammelten Blutes beinhaltet; und
ein Blutbehandlungsgerät (102), das mit dem Auslass (6) des Blutbehandlungsfilters (1) in Verbindung steht, beinhaltet einen sekundären Beutel (107) für die Aufnahme der verbleibenden Blutbestandteile, welche aus dem Auslass (6) fließen und welche durch die Entfernung der vorbestimmten Blutbestandteile erzeugt werden.

**7.** Blutbehandlungskreislauf (100) nach Anspruch 6, wobei das Blutaufnahmegerät (101) umfasst:

den primären Beutel (103);
eine Blutaufnahmenadel (104);
eine erste Röhre (105), die ein mit dem primären Beutel (103) verbundenes Ende und das andere, mit der Blutaufnahmenadel (104) verbundene, Ende beinhaltet; und
eine zweite Röhre (106), die ein mit dem primären Beutel (103) verbundenes Ende und das andere, mit dem Einlass (4) des Blutbehandlungsfilters (1) verbundene, Ende beinhaltet.

**8.** Blutbehandlungskreislauf nach Anspruch 6, wobei der Blutbehandlungsapparat (102) umfasst:

den sekundären Beutel (107);
eine dritte Röhre (110), die ein mit dem Auslass (6) des Blutbehandlungsfilters (1) verbundenes Ende und das andere, mit dem sekundären Beutel (107) verbundene, Ende beinhaltet;
einen dritten Beutel (108) für die Aufbewahrung der von den im sekundären Beutel (107) aufbewahrten Blutzellbestandteile abgetrennten Blutzellbestandteile;
einen vierten Beutel (109), in den vorher eine medizinische Flüssigkeit eingefüllt wurde;
eine vierte Röhre (111), die ein mit dem sekundären Beutel (107) verbundenes Ende beinhaltet;
eine fünfte Röhre (112), die ein mit der vierten Röhre (111) verbundenes Ende und das andere, mit dem dritten Beutel (108) verbundene, Ende beinhaltet; und
einen sechste Röhre (113), die ein mit dem vierten Schlauch (111) verbundenes Ende und das andere, mit dem vierten Beutel (109) verbundene, Ende beinhaltet.

**Revendications**

**1.** Filtre de traitement du sang (1) comprenant :

un boîtier (2) comportant une entrée (4) et une sortie (6), et
une unité de filtration (7) qui est logée dans le boîtier (2) et filtre le sang contenant des érythrocytes, des plaquettes, et des leucocytes comme composants sanguins, dans lequel

l'unité de filtration (7) comprend une unité de filtration principale (11) pour éliminer sensiblement les leucocytes du sang et faire passer les autres composants sanguins au travers, et une unité de pré-filtration (10) qui fait passer les composants sanguins au travers, et

l'unité de filtration principale (11) a un taux d'élimination de 85 à 99% pour les premières microparticules ayant une taille moyenne de particule de 3 $\mu$m qui sont contenues dans une suspension qui la traverse par gravité de sorte qu'une vitesse d'écoulement moyenne de la suspension est de 50 ml/min, et la valeur absolue du potentiel zêta en surface de l'unité de filtration principale (11) est de -2,0 à +2,0 mV
l'unité de pré-filtration (10) a un taux de perméabilité de 70 à 90% pour les microparticules ayant une taille moyenne de particule de 6 $\mu$m qui sont contenues dans la suspension qui la traverse par gravité de sorte qu'une vitesse d'écoulement moyenne de la suspension est de 50 ml/min, et la valeur absolue du potentiel zêta en surface de l'unité de pré-filtration (10) est de -2,0 à +2,0 mV, et
l'unité de filtration (7) a une quantité de sang traité par unité de volume de 10 à 20 ml/cm$^3$ pour le sang qui la traverse par gravité.

**2.** Filtre de traitement du sang selon la revendication 1, dans lequel
un rapport de volume entre l'unité de pré-filtration (10) et l'unité de filtration principale (11) est de 1:5 à 1:20.

**3.** Filtre de traitement du sang (1) selon la revendication 1, dans lequel
l'unité de filtration principale (11) et l'unité de pré-filtration (10) sont des membranes poreuses de type éponge ayant un certain nombre de micro-orifices de communication communiquant avec une face de la membrane et avec l'autre face, et une structure perméable au fluide.

4. Filtre de traitement du sang (1) selon la revendication 1, dans lequel
le sang est traité dans les 24 heures qui suivent le prélèvement de sang chez un donneur.

5. Filtre de traitement du sang (1) selon la revendication 1, dans lequel
une valeur absolue du potentiel zêta en surface de l'unité de filtration principale (11) est de +0,5 à +1,0 mV, et une valeur absolue du potentiel zêta en surface de l'unité de pré-filtration (10) est de +0,5 à +1,0 mV.

6. Circuit de traitement du sang (100) comprenant :

le filtre de traitement du sang (1) selon la revendication 1 ;
un dispositif de prélèvement de sang (101) qui communique avec l'entrée (4) du filtre de traitement du sang (1) et comporte une poche primaire (103) pour stocker le sang prélevé ; et
un dispositif de traitement du sang (102) qui communique avec la sortie (6) du filtre de traitement du sang (1) et comporte une poche secondaire (107) pour collecter les composants du sang restants qui s'écoulent hors de la sortie (6) et qui sont obtenus par l'élimination des composants du sang prédéterminés.

7. Circuit de traitement du sang (100) selon la revendication 6, dans lequel le dispositif de prélèvement de sang (101) comprend :

la poche primaire (103) ;
une aiguille de prélèvement de sang (104) ;
un premier tube (105) comprenant une extrémité reliée à la poche primaire (103) et l'autre extrémité reliée à l'aiguille de prélèvement de sang (104) ; et
un second tube (106) comprenant une extrémité reliée à la poche primaire (103) et l'autre extrémité reliée à l'entrée (4) du filtre de traitement du sang (1).

8. Circuit de traitement du sang selon la revendication 6, dans lequel le dispositif de traitement du sang (102) comprend :

la poche secondaire (107) ;
un troisième tube (110) comprenant une extrémité reliée à la sortie (6) du filtre de traitement du sang (1) et l'autre extrémité reliée à la poche secondaire (107) ;
une troisième poche (108) pour stocker les composants des globules sanguins séparés des composants des globules sanguins stockés dans la poche secondaire (107) ;
une quatrième poche (109) qui est remplie au préalable d'un liquide médical ;
un quatrième tube (111) ayant une extrémité reliée à la poche secondaire (107) ;
un cinquième tube (112) ayant une extrémité reliée au quatrième tube (111) et l'autre extrémité reliée à la troisième poche (108) ; et
un sixième tube (113) ayant une extrémité reliée au quatrième tube (111) et l'autre extrémité reliée à la quatrième poche (109).

# FIG.1

## FIG.2

1

12

4

14

13

3 COVER ⎫
5 BOTTOM ⎬ 2 HOUSING

8

10 PRE-FILTER UNIT ⎫
11 MAIN FILTER UNIT ⎬ 7 FILTER UNIT

9

14

13

12

6

## FIG.3

EP 2 055 329 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001129078 A
- JP 2001198213 A
- US 2001037078 A1
- US 4701267 A
- EP 0630675 A1
- JP H9154920 A
- JP 2002308688 A